# EUROPÄISCHE PATENTSCHRIFT

(11) **EP 4 272 628 B1**
(45) Veröffentlichungstag und Bekanntmachung des Hinweises auf die Patenterteilung: **10.12.2025**
(21) Anmeldenummer: 23020206.1
(22) Anmeldetag: 03.05.2023
(51) Int. Cl.: A61B 5/00, A61B 5/08, A61B 5/087, A61B 5/22, A41D 1/00, A41D 13/12

(54) **KÖRPERTRAGESYSTEM FÜR EINE MOBILE VORRICHTUNG**
BODY SUPPORT SYSTEM FOR A MOBILE DEVICE
SYSTÈME DE SUPPORT DE CORPS POUR DISPOSITIF MOBILE

(30) Priorität: 04.05.2022 DE 102022111060
(43) Veröffentlichungstag der Anmeldung: 08.11.2023
(73) Patentinhaber: Cortex Biophysik GmbH, 04356 Leipzig (DE)
(72) Erfinder: Günther, Andreas, 04158 Leipzig (DE); Siepmann, Markus, 45473 Mülheim an der Ruhr (DE)
(74) Vertreter: Dr. Völger, Wolfgang

(56) Entgegenhaltungen:
- DE-U1- 202007 001 821
- DE-U1- 202014 103 548
- KR-A- 20050 033 932
- US-A1- 2008 091 097

## Beschreibung

Die vorliegende Erfindung betrifft ein Körpertragesystem für eine mobile Vorrichtung, das insbesondere in der Spiroergometrie einsetzbar ist.

Bei dem diagnostischen Verfahren der Spiroergometrie (auch als Ergospirometrie oder Ergospirographie bezeichnet) werden durch die Messung von Atemgasen während einer körperlichen Belastung die Reaktionen von Herz, Kreislauf, Atmung, Stoffwechsel und/oder die kardiopulmonale Leistungsfähigkeit qualitativ und quantitativ untersucht. Diese Untersuchung ist nicht nur unter stationären Bedingungen interessant, wie beispielsweise in einer Arztpraxis, sondern auch insbesondere im Freien, wo reale Belastungsbedingungen beim Laufen, Bergsteigen, Radfahren und dergleichen herrschen.

Für die mobile Spiroergometrie im Freien muss jedoch ein entsprechend geeignetes tragbares Gerät verwendet werden, das zunächst einmal der Anforderung einer geringen Größe und damit eines geringen Gewichts unterliegt, damit es bei der Belastung nicht oder nur in sehr geringem Umfang stört. Solche Geräte sind grundsätzlich bekannt, haben aber aufgrund dieser Vorgaben häufig nur einen sehr begrenzten praktischen Nutzbereich.

Während mobiler Anwendungen im Freien wirken, je nach Anwendungsart (Laufen, Radfahren, Rudern etc.) starke und teilweise sehr unterschiedliche Beschleunigungskräfte auf ein tragbares Gerät. Entsprechend müssen die Tragevorrichtung und die Gerätebefestigung am Probanden diese Kräfte aufnehmen können, ohne unkomfortabel und damit einengend zu wirken. Zudem ist es für die verschiedenen Anwendungsarten notwendig, das tragbares Gerät flexibel am Körper positionieren zu können, um den Bewegungsablauf je nach Sportwart bzw. Belastung nicht einzuschränken.

Bisherige Lösungsansätze aus dem Stand der Technik sehen für das Mitführen eines tragbaren Geräts beispielsweise Tragetaschen vor, welche jedoch in der Positionierung recht unflexibel sind und einen nicht geringen Aufwand bei der Verstauung der tragbaren Geräte sowie der elektrischen und pneumatischen Zuleitungen haben. Alternativen bilden unter anderem starre Metallgerüste, die noch weniger flexibel in der Positionierung des tragbaren Geräts sind und fast ausschließlich ein Mitführen auf dem Rücken erlauben. Zudem benötigen diese Alternativen einen hohen Aufwand bei der Befestigung (z.B. durch Schraubverbindungen). Ein weiterer Lösungsansatz zum Mitführen eines tragbaren Geräts ist bekannt aus DE202007001821.

Angesichts des vorstehend dargelegten Standes der Technik besteht der Bedarf an einer flexibel einsetzbaren Lösung zum Mitführen von tragbaren Geräten.

Der vorliegenden Erfindung liegt daher die Aufgabe zugrunde, ein neuartiges Körpertragesystem für eine mobile Vorrichtung bereitzustellen, das flexibel in der Handhabung ist, eine flexible Positionierung der mobilen Vorrichtung ermöglicht, bei unterschiedlich intensiven Aktivitäten ohne zu stören eingesetzt werden kann und dabei eine mobile Vorrichtung sicher am Körper mitführen lässt.

Diese Aufgabe wird in der vorliegenden Erfindung durch ein Körpertragesystem (5) für eine mobile Vorrichtung (1) gelöst, umfassend
- ein westenartiges Trageelement (51), das ein Rückenteil (511), zwei an das Rückenteil (511) anschließende Schulterträger (53a, 53b) und einen an das Rückenteil (511) anschließenden Hüftgurt (55) aufweist, und
- ein zweiteilig ausgebildetes Halteelement (7) für die mobile Messvorrichtung, dessen beide Teilelemente (7a, 7b) einerseits reversibel an dem westenartigen Trageelement (51) befestigbar und andererseits reversibel miteinander verbindbar sind,

wobei die Schulterträger (53a, 53b) in ihrem vorderen Bereich jeweils Kedern (57) aufweisen,
   und/oder
wobei auf dem Rückenteil (511) des westenartigen Trageelements (51) voneinander beabstandete Kedern (57) vorgesehen sind,
und wobei an den beiden Teilelementen (7a, 7b) des zweiteilig ausgebildeten Halteelements (7) jeweils Kederführungen (71a, 71b) vorgesehen sind, so dass die Kederführungen (71a, 71b) mit den Kedern (57) in Eingriff bringbar sind.

Mit dem Begriff "westenartiges Trageelement" wird im Sinne der vorliegenden Erfindung eine speziell ausgestaltete Weste bezeichnet, die wie eine solche angezogen werden kann. Darüber hinaus umfasst diese speziell ausgestaltete Weste die weiteren, im Folgenden noch zu erläuternden Elemente.

Der Begriff "Keder" ist grundsätzlich bekannt und bezeichnet meist die Randverstärkung eines Tuchs oder einer Plane. Der Keder hat in der Regel einen runden Durchmesser und ist dazu ausgelegt, das als sog. "Kederfahne" angesetzte Tuch in einer sog. Kederschiene zu halten und damit eine Verbindung herzustellen.

Bei dem erfindungsgemäßen Körpertragesystem (5) sind in Abweichung der üblichen Verwendung die Kedern (57) eng und fest mit dem westenartigen Trageelement (51) verbunden.

Die "Kederführungen" sind gemäß der vorliegenden Erfindung ähnlich der bekannten Kederschienen gestaltet und können auf die Kedern aufgeschoben werden. Die mit den Kederführungen (71a, 71b) versehenen Teilelemente (7a, 7b) des zweiteilig ausgebildeten Halteelements (7) sind im vorliegenden Fall gegenüber dem erfindungsgemäßen Körpertragesystem (5) beweglich ausgestaltet.

Die vorliegende Erfindung weist ganz allgemein die Vorteile auf, dass das erfindungsgemäße Körpertragesystem (5) eine Art funktionelles Kleidungsstück darstellt, das eine flexible und gleichzeitig sichere Befestigung der mobilen Vorrichtung (1) unterschiedlich intensiven körperlichen Aktivitäten sicherstellt. Die mobile Vorrichtung (1) kann somit sicher am Körper mitgeführt werden, ohne den Träger wesentlich zu stören oder zu behindern. Das Vorsehen von Kedern (57) auf der Vorderseite als auch auf der Rückseite des erfindungsgemäßen Körpertragesystems (5) erhöht die Variabilität der Erfindung in Bezug auf die Positionierung der mobilen Vorrichtung weiter.

Das minimalistische und durchdachte Design und die intelligent integrierten Merkmale geben dem Probanden die Möglichkeit, das erfindungsgemäße Körpertragesystem (5) in kürzester Zeit eigenständig und ohne fremde Hilfe anzulegen und wieder auszuziehen. Darüber hinaus kann das erfindungsgemäße Körpertragesystem (5) in unterschiedlichen Konfektionsgrößen angeboten werden.

Darüber hinaus ist das erfindungsgemäße Körpertragesystem (5) mit einem Gewicht von etwa 600 g (etwas abhängig von der tatsächlichen Konfektionsgröße) sehr leicht und stellt keine wesentliche Gewichtsbelastung für den Probanden dar.

Ein speziell bevorzugtes Merkmal der Kederführungen (71a, 71b) ist, dass deren Innenprofil konisch ausgelegt ist. Durch den größeren Konusdurchmesser wird das Auffädeln auf die Kedern (57) erleichtert, während auf der anderen Seite der kleinere Konusdurchmesser für eine erhöhte Reibung zwischen Keder (57) und Kederführung (71a, 71b) sorgt, so dass die "Rutschfestigkeit" verstärkt wird.

In einer vorteilhaften Weiterbildung stehen die Kederführungen (71a, 71b) der beiden Teilelemente (7a, 7b) des zweiteilig ausgebildeten Halteelements (7) in der mit dem westenartigen Trageelement (51) verbundenen Position in einem verkippten Winkel zu den Kedern (57).

Mit anderen Worten ist bei diesem speziell bevorzugten Merkmal der Kederführungen (71a, 71b) deren Achse gegenüber der Mittelachse des zweiteilig ausgebildeten Halteelements (7) um einen Winkel größer 0° bis 15° verkippt, was die Reibung zwischen Keder (57) und Kederführung (71a, 71b) und damit die "Rutschfestigkeit" noch weiter erhöht.

Durch die Merkmalskombinationen aus Kedern (57) und Kederführungen (71a, 71b) wird eine stufenlose Höhenverstellbarkeit des zweiteilig ausgebildeten Halteelements (7) und damit der mobilen Vorrichtung (1) entlang der Kedern (57) ermöglicht. Diese kann daher je nach Anwendungsbereich, Sportart, Körpergröße etc. optimal positioniert werden.

In einer bevorzugten Ausführungsform des erfindungsgemäßen Körpertragesystems (5) sind die beiden Teilelemente (7a, 7b) des zweiteilig ausgebildeten Halteelements (7) mittels einer Hakenverbindung (73) formschlüssig verbindbar und bilden eine feste Verschalung zur Aufnahme der mobilen Vorrichtung (1).

Insbesondere wird die Hakenverbindung (73) durch eine vom Körper des Trägers wegweisende Bewegung geschlossen und verriegelt, also als feste Verschalung. Bei eingesetzter mobiler Vorrichtung (1) kann die Hakenverbindung (73) - weder absichtlich noch unabsichtlich - gelöst werden. Diese zweiteilige Ausbildung des Halteelements (7) hat ferner den Vorteil, dass die Teilelemente (7a, 7b) einfacher auf die Kedern (57) aufgefädelt werden können, als es bei einem einteiligen Halteelement der Fall wäre.

Eine andere Ausführungsform sieht vor, dass die Kedern (57) und das zweiteilig ausgebildete Halteelement (7) so angeordnet sind, dass die mobile Vorrichtung (1) in Bezug auf den Körper achsenmittig und / oder körpernah positionierbar ist.

Die Merkmalskombinationen führen zu den Vorteilen, dass zunächst durch die achsenmittige Positionierung durch das Gewicht der mobilen Vorrichtung (1) keine Gewichtsasymmetrie entsteht, welche den Träger in den Bewegungsabläufen behindern kann. Die körpernahe Positionierung trägt zusammen mit der sehr geringen Aufbauhöhe des zweiteilig ausgebildeten Halteelements (7) dazu bei, selbst bei starker Bewegung nur minimale Drehmomente zu verursachen. Auch hierdurch werden Behinderung bei Bewegungsabläufen verringert und die Gefahr des Verrutschens der mobilen Vorrichtung (1) minimiert.

Da viele Sportarten, bei denen das erfindungsgemäße Körpertragesystem (5) Verwendung findet, sehr bewegungsintensiv sind, hat es sich als vorteilhaft herausgestellt, wenn das westenartige Trageelement (51) ferner einen verschließbaren Brustgurt (59) aufweist, der auf der Höhe der Kedern (57) vorgesehen ist. Damit wird gewährleistet, dass die Schulterträger (53a, 53b) nicht bei starken Bewegungen von den Schultern herunterrutschen.

Ein weiteres Merkmal, das zusätzlich zum guten und engen Sitz des erfindungsgemäßen Körpertragesystems (5) beiträgt, ist, dass der Hüftgurt (55) an seinem offenen Ende mit einer verstellbaren Gürtelschnalle (551) versehen ist, welche mit einem magnetischen Verschlusssystem verschließbar ist. Das magnetische Verschlusssystem hat den zusätzlichen Vorteil, dass es leicht und auch einhändig geöffnet werden kann.

Schließlich hat es sich für den festen Sitz und den Tragekomfort des erfindungsgemäßen Körpertragesystems (5) als sehr positiv herausgestellt, wenn die Schulterträger (53a, 53b) jeweils mittels eines verstellbaren und/oder elastischen Gurtbands (531) an den Enden des Hüftgurts (55) befestigt sind.

Wenn die mobile Vorrichtung (1) beispielsweise für die Spiroergometrie ausgelegt ist, sind Kabel und Schläuche zum Anschluss einer Atemmaske und weiterer Komponenten notwendig. Um diese zu verstauen und zu befestigen, sind bevorzugt an den Schulterträgern (53a, 53b) Befestigungslaschen (535) zur Aufnahme von Kabeln und Schläuchen der mobilen Vorrichtung (1) vorgesehen. Hiermit wird auch ein Abknicken der Schläuche verhindert. Da die Befestigungslaschen (535) bevorzugt mit einem Magnetverschluss versehen sind, ist zudem ein leichtes einhändiges Lösen möglich.

Eine andere spezielle Weiterbildung sieht vor, dass das Rückenteil (511) eine Anordnung von Polstern und Netzelementen aufweist und ferner zumindest eine Tasche (513) zu Aufnahme von Gegenständen vorgesehen ist. Polster und Netzelemente erhöhen den Tragekomfort des erfindungsgemäßen Körpertragesystems (5). Die eingearbeitete Tasche kann beispielsweise Ersatzakkus für die mobile Vorrichtung (1) oder Überlängen von Kabeln und Schläuchen aufnehmen.

Ein zweiter Aspekt der vorliegenden Erfindung betrifft ein mobiles System zur Erfassung von Atemgasbestandteilen, umfassend
- ein Körpertragesystem (5), wie es vorstehend beschrieben wurde, und
- eine mobile Vorrichtung (1),
wobei das mobile System für die mobile Leistungsdiagnostik in der Spiroergometrie ausgelegt ist.

Aufgrund des am erfindungsgemäßen Körpertragesystem (5) vorgesehenen zweiteilig ausgebildeten Halteelements (7) kann die mobile Vorrichtung (1) sehr schnell und einfach angebracht und abgenommen werden.

Wie vorstehend schon angegeben, kommt das erfindungsgemäße Körpertragesystem (5) auf ein Gewicht von etwa 600 g, während die mobile Vorrichtung (1) (inklusive Batterien) etwa 580g wiegt. Das mobile System zur Erfassung von Atemgasbestandteilen hat damit ein Gesamtgewicht von lediglich rund 1.200 g.

Weitere Ziele, Merkmale, Vorteile und Anwendungsmöglichkeiten ergeben sich aus der nachfolgenden Beschreibung.

Es zeigen:
- Fig. 1: eine grafische Darstellung der Vorderseite eines westenartigen Trageelements 51 nach einer bevorzugten Ausführungsform der Erfindung,
- Fig. 2: eine grafische Darstellung der Rückseite 511 eines westenartigen Trageelements 51 nach einer bevorzugten Ausführungsform der Erfindung
- Fig. 3: eine grafische Darstellung des zweiteilig ausgebildeten Halteelements 7 nach einer bevorzugten Ausführungsform der Erfindung,
- Fig. 4: eine schematische Darstellung des erfindungsgemäßen Körpertragesystems 5 nach einer bevorzugten Ausführungsform der Erfindung mit vorderseitig angeordnetem zweiteilig ausgebildeten Halteelement 7,
- Fig. 5: eine schematische Darstellung des erfindungsgemäßen Körpertragesystems 5 nach einer bevorzugten Ausführungsform der Erfindung mit rückseitig angeordnetem zweiteilig ausgebildeten Halteelement 7,
- Fig. 6: eine schematische Darstellung mobilen Systems zur Erfassung von Atemgasbestandteilen nach einer bevorzugten Ausführungsform der Erfindung mit erfindungsgemäßem Körpertragesystem 5 und daran angebrachter mobiler Vorrichtung 1 und
- Fig. 7: eine grafische Darstellung des Verriegelungssystems an der mobilen Vorrichtung 1 nach einer Ausführungsform der Erfindung.

In den Figuren werden alle gleichen Bauteile mit den gleichen Bezugszeichen benannt, aus Gründen der Übersichtlichkeit sind aber nicht unbedingt in allen Darstellungen alle Bezugszeichen eingefügt.

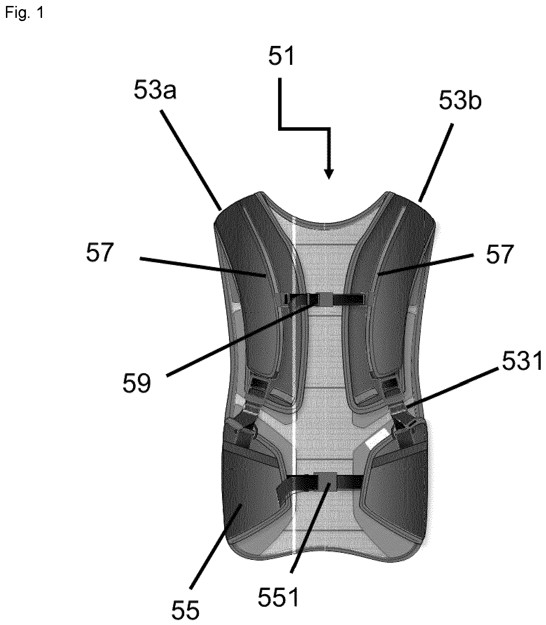

Figur 1 zeigt eine grafische Darstellung der Vorderseite eines westenartigen Trageelements 51 nach einer bevorzugten Ausführungsform der Erfindung, bei welcher die Schulterträger 53a, 53b jeweils mit einer Keder 57 versehen sind. Die Schulterträger 53a, 53b sind jeweils mit einem elastischen Gurtband 531 an den Enden des Hüftgurts 55 befestigt, der mittels einer verstellbaren Gürtelschnalle 551 verschließbar ist.

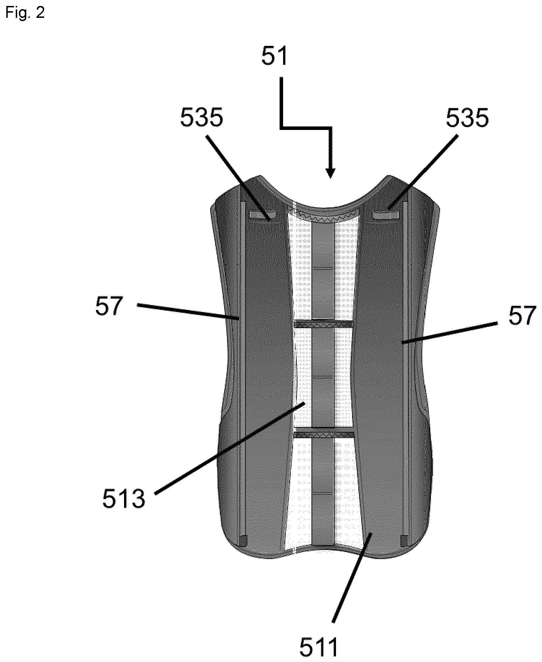

Die Rückseite 511 des westenartigen Trageelement 51 wird schematisch in Figur 2 gezeigt. In dieser Ausführungsform sind zwei Kedern 57 voneinander beabstandet angeordnet. Dazwischen befindet sich eine Tasche 513. Im oberen Bereich der Rückteils 511 sind Befestigungslaschen 535 zur Aufnahme von Kabeln und Schläuchen vorgesehen.

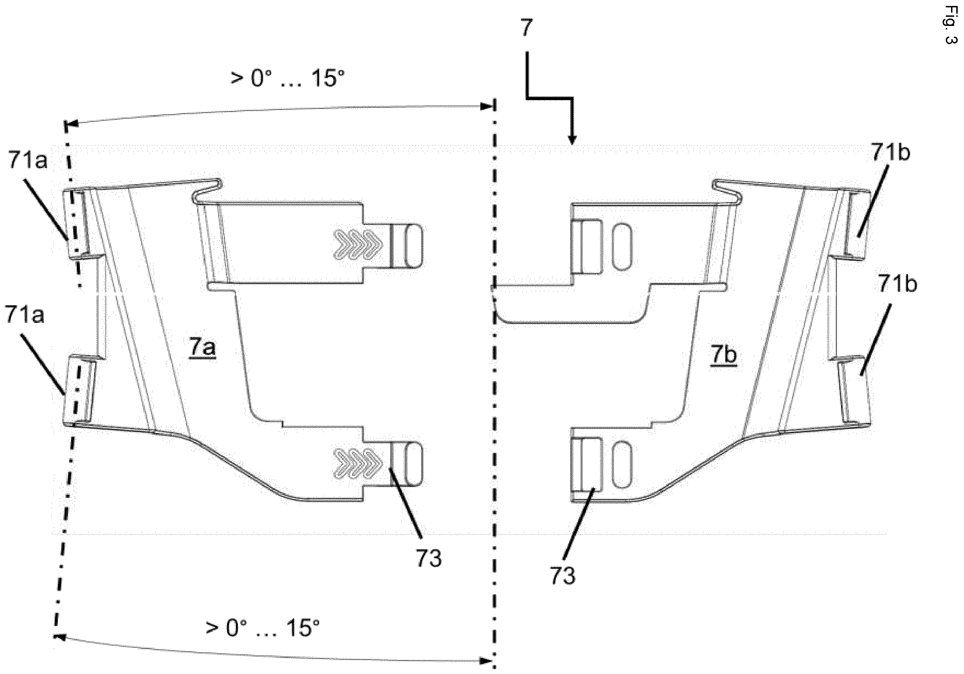

Figur 3 zeigt im Detail eine grafische Darstellung des zweiteilig ausgebildeten Halteelements 7 nach einer bevorzugten Ausführungsform der Erfindung, auf dessen Funktion nachstehend noch eingegangen wird. In dieser Darstellung sind für jedes der beide Teilelemente 7a, 7b jeweils zwei Kederführungen 71a, 71b vorgesehen, die auf die Kedern 57 auffädelbar sind. In dieser Darstellung ist der verkippte Winkel der Kederführungen 71a, 71b gegenüber der Mittelachse deutlich zu erkennen, er liegt bei etwas mehr als 0° bis 15°.

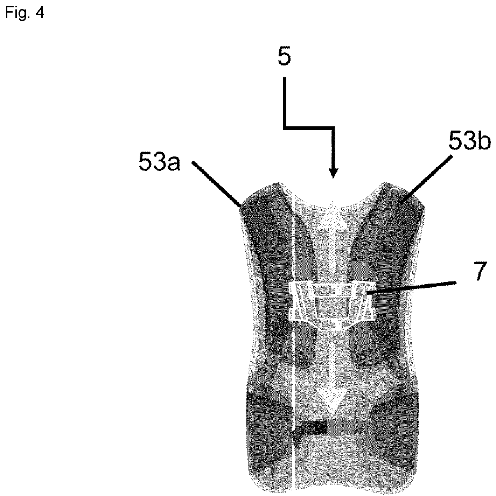

Figur 4 stellt schematisch dar, wie bei dem erfindungsgemäßen Körpertragesystem 5 nach einer bevorzugten Ausführungsform der Erfindung das zweiteilig ausgebildete Halteelement 7 auf der Vorderseite angeordnet ist. Die Pfeile deuten an, wie sich das Halteelement 7 flexibel in der Höhe positionieren lässt.

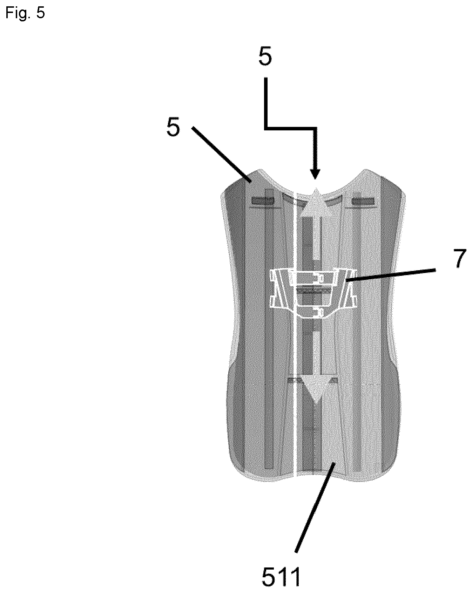

In ähnlicher Weise zeigt Figur 5 schematisch, wie bei dem erfindungsgemäßen Körpertragesystem 5 nach einer bevorzugten Ausführungsform der Erfindung das zweiteilig ausgebildete Halteelement 7 auf der Vorderseite angeordnet ist, wobei die Pfeile wieder andeuten, wie sich das Halteelement 7 flexibel in der Höhe auch auf dem Rücken positionieren lässt.

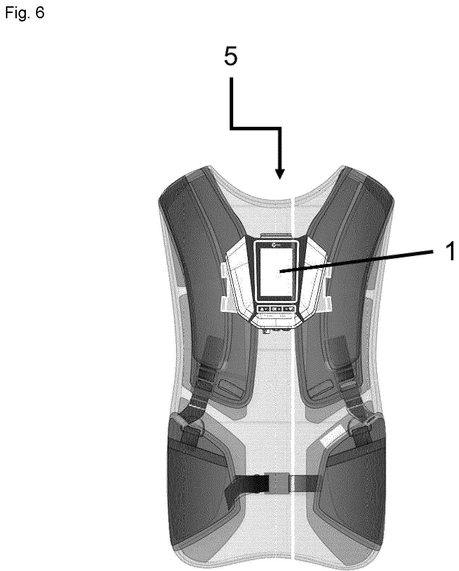

Eine schematische Darstellung mobilen Systems zur Erfassung von Atemgasbestandteilen nach einer bevorzugten Ausführungsform der Erfindung ist der Figur 6 zu entnehmen. An dem erfindungsgemäßem Körpertragesystem 5 ist mittels Halteelement 7 eine mobile Vorrichtung 1 angebracht, in dieser Darstellung auf der Vorderseite, d.h. auf der Brust des Probanden.

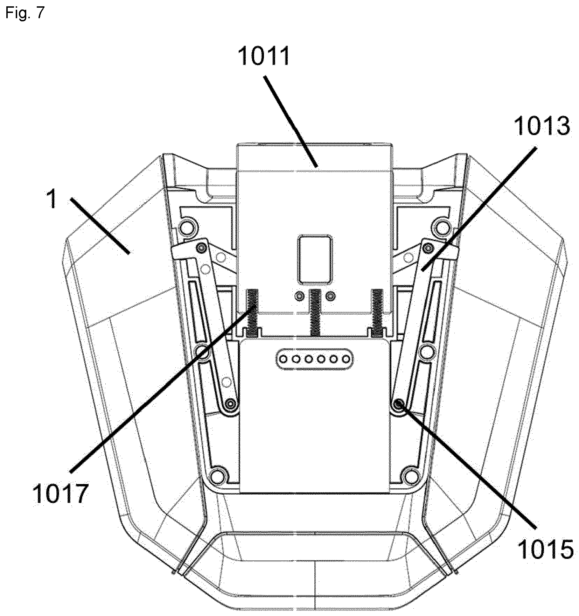

Figur 7 zeigt schließlich schematisch, wie das Verriegelungssystem an der mobilen Vorrichtung 1 nach einer Ausführungsform der Erfindung ausgebildet ist, so dass diese auch einhändig angebracht und entfernt werden kann.

Nachstehend wird eine bevorzugte, die vorliegende Erfindung aber nicht einschränkende Ausführungsform beschrieben. Die vorliegende Erfindung wird durch die Ansprüche definiert.

Im Zusammenhang mit der Entwicklung eines neuartigen Systems zur Erfassung von Atemgasbestandteilen, das insbesondere in der Spiroergometrie einsetzbar ist, wurde der Bedarf an einem neuen funktionalen Tragesystem für Probanden ermittelt, welches mit der neu konzipierten mobilen Vorrichtung 1 spiroergometrische Messungen bzw. Leistungsdiagnostik in der mobilen Anwendung (z. B. Feldtest oder Labor) ermöglicht.

Dabei sollte das neue Tragesystem ein Maximum an Komfort und Bewegungsfreiheit für den Probanden bieten und allen Ansprüchen der mobilen Leistungsdiagnostik, unabhängig des spezifischen Anwendungsbereiches, gerecht werden. Ein weiteres besonderes Augenmerk lag in der intuitiven und einfachen Bedienung des Tragesystems in Zusammenspiel (einfache Integration) mit der mobilen Vorrichtung 1 unter Berücksichtigung einer optimalen Gewichtsverteilung für den Probanden und mit einer flexiblen Positionierung der mobilen Vorrichtung 1 am Körper des Probanden.

Das in den Figuren dargestellte erfindungsgemäße Körpertragesystem 5 nach dieser speziellen Ausführungsform weist auf der Vorderseite (Brustpartie) zwei wesentliche funktionale Einzelelemente auf, die Schulterträger 53a, 53b und den Hüftgurt 55. Optional kann ein Brustgurt 59 vorgesehen sein.

Die ergonomisch geformten Schulterträger 53a, 53b verbessern im Vergleich zu bisher bekannten Tragsystemen im Bereich der Spiroergometrie die Bewegungsfreiheit des Probanden bei gleichzeitiger Erhöhung des Tragekomforts. Diese sind vorzugsweise mit einem elastischen Gurtband 531 (inklusive D-Ring) jeweils links und rechts an den Enden des Hüftgurtes 55 verbunden und lassen sich zur besseren Ausrichtung auf die Körpergröße des Probanden der Länge nach verstellen. Dadurch wird gewährleistet, dass die Schulterpolster der Schulterträger 53a, 53b ergonomisch auf den Schultern aufliegen. Als zusätzliche Komponente sind im oberen Bereich der Schulterträger Befestigungslaschen 535 für die Kabel und Schläuche der Gas-Absaugstrecke bzw. den Volumenstromsensor vorhanden. Speziell handelt es sich hierbei um magnetische Schlaufen, die sich leicht vom Probanden selbst befestigen und lösen lassen. Durch diese flexiblen Vorrichtungen wird ein Abknicken/Quetschen der Kabel/Schläuche verhindert, überschüssige Kabellängen sind so direkt verstaubar und können individuell justiert werden, je nachdem, ob sich die mobile Vorrichtung 1 im Brust- oder Rückenbereich befindet.

Als wesentliches funktionales Element sind die Schulterträger jeweils mit Kedern 57 versehen. Diese dienen nicht nur zur zusätzlichen Formgebung der Schulterträger 53a, 53b, sondern fungieren hauptsächlich als Befestigung für das zweiteilig ausgebildete Halteelement 7 der mobilen Vorrichtung 1. Mit ihnen ist eine nahezu freie vertikale Positionierung im Brustbereich möglich.

Der mehrschichtig gepolsterte, flexibel und anatomisch geformte Hüftgurt 55 bietet einen festen, angenehmen Sitz auf den Beckenknochen und überträgt das gesamte Gewicht der mobilen Vorrichtung 1 auf die Hüften während der bewegungsintensiven Sportart bzw. des Messvorgangs. Der Hüftgurt 55 ist insbesondere bevorzugt mit einer Fidlock-Gürtelschnalle (verstellbare Gürtelschnalle 551) versehen, welche mit einem magnetischen Verschlusssystem funktioniert, um ein schnelles Anlegen bzw. Ablegen des erfindungsgemäße Körpertragesystems 5 eigenständig durch den Probanden zu ermöglichen.

Die an den Kedern 57 fixierbaren (optionalen) Brustgurte 59 sind in der Höhe und Breite verstellbar und verhindern, dass die Schulterträger 53a, 53b seitlich verrutschen. In Verbindung mit der mobilen Vorrichtung 1 wird so ein besserer Tragekomfort unter Berücksichtigung einer gleichmäßigen Gewichtsverteilung (Schwerpunkt nah am Körperschwerpunkt des Probanden) gesichert.

Durch das nahtlos gestickte Rückenteil 511 und die spezielle Platzierung von Polstern aus Schaumstoff und Mesh werden die Druckverteilung und auch die Belüftung optimiert und somit ein maximaler Tragekomfort erzeugt. Ergänzend wurden auf der Rückseite des erfindungsgemäße Körpertragesystems 5 drei zusätzliche Taschen mit Verklemmungen integriert, um Ausrüstung (z. B. Smartphone etc.) schnell und einfach zu verstauen.

Des Weiteren befinden sich ebenfalls auf der Rückseite des erfindungsgemäßen Körpertragesystems 5 Kedern 57, die eine flexible Positionierung der mobilen Vorrichtung 1 auch auf dem Rückenteil 511 mit Hilfe des speziell entworfenen zweiteilig ausgebildetes Halteelements 7 sicher ermöglichen.

Eine essenzielle Anforderung an das neue Tragesystem stellte die optimale Positionierung der mobilen Vorrichtung 1 während der mobilen Leistungsdiagnostik dar. Hierbei sollte die mobile Vorrichtung 1 stets optimal an dem Probanden befestigt sein, ohne dass dieser in seiner Bewegungsfreiheit eingeschränkt wird. Außerdem galt es zu berücksichtigen, dass sich der Schwerpunkt der mobilen Vorrichtung 1 immer in Körpernähe (sowohl im Brust- als auch Rückenbereich) des Probanden befindet, um dadurch eine bessere Kontrolle über auftretende Beschleunigungskräfte zu erwirken. Um diesen Anforderungen gerecht zu werden, wurde ein speziell konzipiertes zweiteilig ausgebildetes Halteelement 7 entwickelt. Dieses besteht aus einem linken und einem rechten Teilelement 7a, 7b und kann in zusammengesetzter Form als eine Art Verschalung verstanden werden.

Um das zweiteilig ausgebildete Halteelement 7 optimal in das erfindungsgemäße Körpertragesystem 5 zu integrieren, muss zunächst das entsprechende Teilelement 7a, 7b, welches mit einer speziellen Kederführung 71a, 71b versehen ist, a) auf dem linken und dem rechten Schulterträger 53a, 53b eingeführt bzw. b) auf der Rückenpartie 511 des erfindungsgemäßen Körpertragesystems 5 ebenfalls auf der linken und rechten Seite eingeschoben werden. Anschließend werden die beiden Teilelemente 7a, 7b mit einer Hakenverbindung 73 formschlüssig verbunden. Dieses als Verschalung gestaltete Halteelement 7 garantiert, dass keine Druckstellen am Körper des Probanden entstehen können. Durch die Steifigkeit des Halteelements 7, in Verbindung mit den im Brustbereich gebogenen und im Rückenbereich auseinandergesetzten Kedern 57, wird eine Zugspannung erzeugt, die ein Verrutschen verhindern. Zusätzlich sind die beiden Kederführungen 71a, 71b, die sich jeweils auf dem rechten und linken Teilelement 7a, 7b des Halteelements 7 befinden, in einem bestimmten Winkel angeordnet, wodurch eine zusätzliche Reibungskraft erzeugt wird, die einem Verrutschen auch bei größeren Kräften entgegenwirkt.

Die mobile Vorrichtung 1 verfügt an der Rückseite über ein spezielles Verriegelungssystem, mit dem diese a) in einer stationäre Vorrichtung 3 (sog. Docking Station) arretiert, als auch b) in dem Halteelement 7 des erfindungsgemäßen Körpertragesystems 5 befestigt werden kann. Dieses Verriegelungssystem wird in Figur 7 schematische dargestellt. Die Einrast-Funktion kann der Proband eigenständig mit einer Hand auslösen. Hierfür wird mit Hilfe des Zeige- und Mittelfingers die "Click & GO" Taste 1011 an der mobilen Vorrichtung 1 nach unten gedrückt. Dies bewirkt, dass die seitlichen Rasthaken 1013 über deren Drehpunkt 1015 nach innen gezogen und der Verschlussmechanismus freigegeben wird (Release-Funktion). Danach kann die mobile Vorrichtung 1 in die Verschalung des Halteelements 7 gelegt werden. Sobald der Proband die "Click & GO" Taste loslässt, werden über einen Federmechanismus 1017 (die Rasthaken 1013 und die "Click & GO" Taste 1011 wieder in die Ausgangsstellung zurückgeführt (Lock-Funktion). Über diese Funktionalität wird gewährleistet, dass die mobile Vorrichtung 1 auch bei möglicher Fremdeinwirkung in dem erfindungsgemäßen Körpertragesystems 5 arretiert bleibt.

### Bezugszeichenliste

- 1: mobile Vorrichtung
- 1011: "Click & GO" Taste
- 1013: Rasthaken
- 1015: Drehpunkt
- 1017: Federmechanismus
- 5: Körpertragesystem
- 51: westenartiges Trageelement
- 511: Rückenteil
- 513: Tasche
- 53a, 53b: Schulterträger
- 531: elastisches Gurtband
- 535: Befestigungslaschen
- 55: Hüftgurt
- 551: Gürtelschnalle
- 57: Keder
- 59: Brustgurt
- 7: Halteelement
- 7a, 7b: Teilelemente
- 71a, 71b: Kederführungen
- 73: Hakenverbindung

## Patentansprüche

1. Körpertragesystem (5) für eine mobile Vorrichtung (1), umfassend
- ein westenartiges Trageelement (51), das ein Rückenteil (511), zwei an das Rückenteil (51) anschließende Schulterträger (53a, 53b) und einen an das Rückenteil (51) anschließenden Hüftgurt (55) aufweist, und
- ein zweiteilig ausgebildetes Halteelement (7) für die mobile Messvorrichtung, dessen beide Teilelemente (7a, 7b) einerseits reversibel an dem westenartigen Trageelement (51) befestigt und andererseits reversibel miteinander verbunden sind,
wobei die Schulterträger (53a, 53b) in ihrem vorderen Bereich jeweils Kedern (57) aufweisen
und/oder
wobei auf dem Rückenteil (511) des westenartigen Trageelements (51) voneinander beabstandete Kedern (57) vorhanden sind,
und wobei an den beiden Teilelementen (7a, 7b) des zweiteilig ausgebildeten Halteelements (7) jeweils Kederführungen (71a, 71b) vorhanden sind, so dass die Kederführungen (71a, 71b) mit den Kedern (57) in Eingriff bringbar sind.

2. Körpertragesystem (5) nach Anspruch 1, wobei die Kederführungen (71a, 71b) der beiden Teilelemente (7a, 7b) des zweiteilig ausgebildeten Halteelements (7) in der mit dem westenartigen Trageelement (51) verbundenen Position in einem verkippten Winkel zu den Kedern (57) stehen.

3. Körpertragesystem (5) nach Anspruch 1 oder 2, wobei die beiden Teilelemente (7a, 7b) des zweiteilig ausgebildeten Halteelements (7) mittels einer Hakenverbindung (73) formschlüssig verbindbar sind und eine feste Verschalung zur Aufnahme der mobilen Vorrichtung (1) aufweist.

4. Körpertragesystem (5) nach einem der Ansprüche 1 bis 3, wobei die Kedern (57) und das zweiteilig ausgebildete Halteelement (7) so angeordnet sind, dass die mobile Vorrichtung (1) in Bezug auf den Körper achsenmittig und / oder körpernah positionierbar ist.

5. Körpertragesystem (5) nach einem der Ansprüche 1 bis 4, wobei das westenartige Trageelement (51) ferner einen verschließbaren Brustgurt (59) aufweist, der auf der Höhe der Kedern (57) vorgesehen ist.

6. Körpertragesystem (5) nach einem der Ansprüche 1 bis 5, wobei der Hüftgurt (55) an seinem offenen Ende mit einer verstellbaren Gürtelschnalle (551) versehen ist, welche mit einem magnetischen Verschlusssystem verschließbar ist.

7. Körpertragesystem (5) nach einem der Ansprüche 1 bis 6, wobei die Schulterträger (53a, 53b) jeweils mittels eines verstellbaren und/oder elastischen Gurtbands (531) an den Enden des Hüftgurts (55) befestigt sind.

8. Körpertragesystem (5) nach einem der Ansprüche 1 bis 7, wobei an den Schulterträgern (53a, 53b) Befestigungslaschen (535) zur Aufnahme von Kabeln und Schläuchen der mobilen Vorrichtung (1) vorhanden sind.

9. Körpertragesystem (5) nach einem der Ansprüche 1 bis 8, wobei das Rückenteil (511) eine Anordnung von Polstern und Netzelementen aufweist und ferner zumindest eine Tasche (513) zu Aufnahme von Gegenständen vorhanden ist.

10. Mobiles System zur Erfassung von Atemgasbestandteilen, umfassend
- ein Körpertragesystem (5) nach einem der Ansprüche 1 bis 9 und
- eine mobile Vorrichtung (1),
wobei das mobile System für die mobile Leistungsdiagnostik in der Spiroergometrie ausgelegt ist.

## Claims

1. Body carrier system (5) for a mobile device (1), comprising
- a vest-like carrying element (51) comprising a back part (511), two shoulder straps (53a, 53b) connected to the back part (51) and a hip belt (55) connected to the back part (51), and
- a two-part holding element (7) for the mobile measuring device, whose both sub-elements (7a, 7b) are reversibly fixed to the vest-like carrying element (51) as well as reversibly connected to each other,
wherein each shoulder strap (53a, 53b) is equipped with welts (57) in the front
and/or
wherein spaced welts (57) are present on the back part (511) of the vest-like carrying element (51),
and wherein on each of the two sub-elements (7a, 7b) of the two-part holding element (7) welt guides (71a, 71b) are present so that the welt guides (71a, 71b) can be engaged with the welts (57).

2. Body carrier system (5) according to claim 1, wherein the welt guides (71a, 71b) of the two sub-elements (7a, 7b) of the two-part support element (7) are at a tilted angle to the welts (57) in the position connected to the vest-like supporting element (51).

3. Body carrier system (5) according to claim 1 or 2, wherein the two sub-elements (7a, 7b) of the two-part support element (7) are connectable in a form-fitting manner by means of a hook connection (73) and comprise a rigid casing for accommodating the mobile device (1).

4. Body carrier system (5) according to one of claims 1 to 3, wherein the welt (57) and the two-part support element (7) are arranged such a way that the mobile device (1) can be positioned axially centred and/or close to the body.

5. Body carrier system (5) according to one of claims 1 to 4, wherein the vest-like carrying element (51) further comprises a closable chest strap (59) which is provided at the height of the welts (57).

6. Body carrier system (5) according to one of claims 1 to 5, wherein the open end of hip belt (55) is provided with an adjustable belt buckle (551) to be closed with a magnetic closure system.

7. Body carrier system (5) according to one of claims 1 to 6, wherein each of the two shoulder straps (53a, 53b) is fastened by an adjustable and/or elastic webbing (531) at the ends of the hip belt (55).

8. Body carrier system (5) according to one of claims 1 to 7, wherein on the shoulder carriers (53a, 53b) fastening lugs (535) are present for holding cables and hoses of the mobile device (1).

9. Body carrier system (5) according to one of claims 1 to 8, wherein the back part (511) has an arrangement of pads and mesh elements and at least one pocket (513) is present for holding objects.

10. Mobile system for detecting respiratory gas components, comprising
- a body carrier system (5) according to one of claims 1 to 9 and
- a mobile device (1),
wherein the mobile system is designed for mobile performance diagnostics in spiroergometry.

## Revendications

1. Système de support corporel (5) pour un dispositif mobile (1), comprenant
- un élément de support (51) de type gilet, qui présente une partie dorsale (511), deux supports d'épaule (53a, 53b) se raccordant à la partie dorsale (51) et une ceinture de hanches (55) se raccordant à la partie dorsale (51), et
- un élément de retenue (7) réalisé en deux parties pour le dispositif de mesure mobile, dont les deux éléments partiels (7a, 7b) sont d'une part fixés de manière réversible à l'élément de support (51) de type gilet et d'autre part reliés entre eux de manière réversible,
les supports d'épaule (53a, 53b) présentant chacun des bourrelets (57) dans leur zone avant
et/ou
des bourrelets (57) espacés les uns des autres étant présents sur la partie dorsale (511) de l'élément de support (51) de type gilet,
et des guides de bourrelets (71a, 71b) étant respectivement présents sur les deux éléments partiels (7a, 7b) de l'élément de retenue (7) réalisé en deux parties, de telle sorte que les guides de bourrelets (71a, 71b) peuvent être mis en prise avec les bourrelets (57).

2. Système de support corporel (5) selon la revendication 1, dans lequel les guides de bourrelet (71a, 71b) des deux éléments partiels (7a, 7b) de l'élément de retenue (7) réalisé en deux parties forment un angle incliné par rapport aux bourrelets (57) dans la position reliée à l'élément de support (51) de type gilet.

3. Système de support corporel (5) selon la revendication 1 ou 2, dans lequel les deux éléments partiels (7a, 7b) de l'élément de retenue (7) réalisé en deux parties peuvent être reliés par complémentarité de forme au moyen d'une liaison à crochet (73) et présente un coffrage fixe pour recevoir le dispositif mobile (1).

4. Système de support corporel (5) selon l'une quelconque des revendications 1 à 3, dans lequel les bourrelets (57) et l'élément de retenue (7) réalisé en deux parties sont agencés de telle sorte que le dispositif mobile (1) peut être positionné de manière axialement centrale et/ou à proximité du corps par rapport au corps.

5. Système de support corporel (5) selon l'une quelconque des revendications 1 à 4, dans lequel l'élément de support (51) de type gilet présente en outre une sangle de poitrine (59) pouvant être fermée, qui est prévue à la hauteur des bourrelets (57).

6. Système de support corporel (5) selon l'une quelconque des revendications 1 à 5, dans lequel la ceinture de hanches (55) est munie à son extrémité ouverte d'une boucle de ceinture réglable (551) qui peut être fermée par un système de fermeture magnétique.

7. Système de support corporel (5) selon l'une quelconque des revendications 1 à 6, dans lequel les supports d'épaule (53a, 53b) sont fixés chacun aux extrémités de la ceinture de hanches (55) au moyen d'une sangle réglable et/ou élastique (531).

8. Système de support corporel (5) selon l'une quelconque des revendications 1 à 7, dans lequel des pattes de fixation (535) sont présentes sur les supports d'épaule (53a, 53b) pour recevoir des câbles et des tuyaux du dispositif mobile (1).

9. Système de support corporel (5) selon l'une quelconque des revendications 1 à 8, dans lequel la partie dorsale (511) présente un agencement de rembourrages et d'éléments en filet et en outre au moins une poche (513) destinée à recevoir des objets est présente.

10. Système mobile pour la détection de composants de gaz respiratoire, comprenant
- un système de support corporel (5) selon l'une quelconque des revendications 1 à 9 et
- un dispositif mobile (1),
le système mobile étant conçu pour le diagnostic de performance mobile en spiroergométrie.
